# EUROPEAN PATENT APPLICATION

(11) **EP 1 902 730 A1**
(43) Date of publication of application: **26.03.2008**
(21) Application number: 06766566.1
(22) Date of filing: 06.06.2006
(51) Int. Cl.: A61K 45/00, A61K 38/00, A61P 1/12, C12Q 1/02, C12Q 1/68, C12N 15/09

(54) **NPY Y2 AGONIST FOR USE AS THERAPEUTIC AGENT FOR DISEASE ACCOMPANIED BY DIARRHEA**

(30) Priority: 09.06.2005 JP 2005169851
(71) Applicant: BANYU PHARMACEUTICAL CO., LTD., Tokyo 102-8667 (JP)
(72) Inventor: SUZUKI, Jun, 3, Okubo, Tsukuba-shi, Ibaraki 3002611 (JP); MORIYA, Ryuichi, 3, Okubo, Tsukuba-shi, Ibaraki 3002611 (JP); HIROSE, Hiroyasu, 3, Okubo, Tsukuba-shi, Ibaraki 3002611 (JP); KANATANI, Akio, 3, Okubo, Tsukuba-shi, Ibaraki 3002611 (JP)
(74) Representative: Buchan, Gavin MacNicol
(86) International application number: PCT/JP2006/311704
(87) International publication number: WO 2006/132406

(57) **Abstract**

An NPY Y2 agonist is used as a therapeutic agent for a disease accompanied by diarrhea. A compound capable of treating the disease can be evaluated and screened by examining the compound on the targeting to an NPY Y2 receptor. An agent capable of inhibiting the diarrhea by a novel mechanism of action; and a method for evaluation of a compound, including the screening for the agent.

## Description

### Technical Field

The present invention relates to a novel use of an NPY Y2 agonist for the treatment of a disease accompanied by diarrhea, and also relates to a method for evaluating a compound which targets NPY Y2 and is effective in the treatment of a disease accompanied by diarrhea.

### Background Art

NPY (neuropeptide Y) is a peptide consisting of 36 amino acids, and was isolated from the porcine brain by Tatemoto et al. in 1982 (Nature, vol. 296, p. 659, 1982: Non-patent document 1). NPY is widely distributed in the central nervous system and peripheral nervous system, and performs a variety of functions in vivo as one of the most abundant peptides in the nervous systems.

That is, NPY acts as an orexigenic substance in the central nervous system, and also promotes markedly the fat accumulation via the mediation of the secretion of various hormones or the action of the nervous systems. For example, it is known that continuous intracerebroventricular administration of NPY induces obesity and insulin resistance on the basis of such actions. It is also known that NPY has central actions such as regulation of depression, anxiety, schizophrenia, pain, dementia and circadian rhythm, etc. Further, NPY coexists with norepinephrine at the sympathetic nerve terminals in the peripheral nervous system, and is related to the tonicity of the sympathetic nerve system. It is known that peripheral administration of NPY causes vasoconstriction and also enhances the actions of other vasoconstrictive substances such as norepinephrine.

NPY has a wide variety of pharmacological actions via the receptors partially shared by its analogs, peptide YY (hereinafter referred to as PYY) and a pancreatic polypeptide (hereinafter referred to as PP). These pharmacological actions of NPY are known to be induced by the action of at least five types of receptors such as NPY Y1 to Y5 either solely or through their mutual interactions. (Trends in Neuroscience, vol. 20, p. 294, 1997: Non-patent document 2).

NPY Y2 receptor was cloned by Rose et al. in 1995 (J. Biol. Chem. vol. 270, p. 22661, 1995: Non-patent document 3; J. Biol. Chem. vol. 270, p. 26758, 1995: Non-patent document 4, etc.). NPY Y2 receptor has a seven membrane spanning structure and shows a homology of 31 % with Y1 receptor at the amino acid level. From the results of Northern analysis, NPY Y2 receptor is strongly expressed in the brain and weakly expressed in the small intestine, however, it is not expressed widely in tissues unlike Y1 receptor. In the brain, while NPY Y2 receptor is strongly expressed particularly in the cerebral cortex and hippocampus, it is hardly expressed in the cerebellum and spinal cord.

The functions of NPY and related peptides are expressed through binding to the NPY receptors present in the central or peripheral nervous system. Therefore, the expression of these pharmacological actions can be prevented by blocking the binding of NPY and related peptides to NPY receptors. In consequence of this, it is expected that a substance which antagonizes the binding of NPY and related peptides to NPY receptors may be useful in the prevention or treatment of various diseases associated with NPY and related peptides, for example, cardiovascular diseases such as angina, acute or congestive heart failure, myocardial infarction, hypertension, renal disease, electrolyte abnormality and vasospasm, nervous system diseases such as bulimia, depression, anxiety, seizure, epilepsy, dementia, pain, alcoholism, withdrawal symptoms accompanying the discontinuance of drug, circadian rhythm disorders, schizophrenia, memory impairment, sleep disorders and cognitive impairment, metabolic diseases, such as obesity, diabetes, impaired hormone secretion, gout and fatty liver, reproductive system diseases such as infertility, preterm delivery and sexual dysfunction, gastrointestinal diseases, respiratory diseases, inflammatory diseases, glaucoma and the like.

In this way, the elucidation of the in vivo functions of NPY and related peptides and their receptors will lead to the development of therapeutic agents or diagnostic agents for the above-mentioned various diseases, therefore, a research for development of a new medicine targeting NPY or NPY receptors has been rapidly advanced.

On the other hand, a disease accompanied by diarrhea such as diarrhea or irritable bowel syndrome (IBS) has become a problem not only as a gastrointestinal disease but also as a chronic disease reflecting the current lifestyle, and a lot of medicines have already been used for suppressing such a symptom. As such medicines, an antispasmodic agent, a lactic acid bacteria preparation, an antimicrobial agent, an antiinflammatory agent, a prokinetic agent and the like are known, and from the viewpoint of the mechanism, a muscarinic antagonist, a serotonin antagonist, a µ-opioid agonist and the like are known (Drug & Aging, vol. 18, p. 201, 2001: Non-patent document 5).
(Non-patent document 1): Nature, vol. 296, p. 659, 1982
(Non-patent document 2): Trends in Neuroscience, vol. 20, p. 294, 1997
(Non-patent document 3): J. Biol. Chem. vol. 270, p. 22661, 1995
(Non-patent document 4): J. Biol. Chem. vol. 270, p. 26758, 1995
(Non-patent document 5): Drug & Aging, vol. 18, p. 201, 2001

### Disclosure of the Invention

However, the present situation is that as an agent for inhibiting diarrhea, an agent based on a new mechanism with less side effects has been demanded.

The present invention has been made in view of the above-mentioned problem of the prior art, and has its object to provide an agent capable of inhibiting diarrhea based on a new mechanism of action. It also has its object to provide a method for evaluating a compound including screening of such an agent.

The present inventors made intensive studies in order to achieve the above objects, and as a result, they found that an NPY Y2 agonist inhibits bowel motility and works to prevent diarrhea, and thus completed the present invention.

That is, the NPY Y2 agonist of the present invention is characterized in that it is a therapeutic agent for a disease accompanied by diarrhea.

Further, the NPY Y2 agonist of the present invention is characterized in that the NPY Y2 agonist is an NPY analog.

Further, the NPY Y2 agonist of the present invention is characterized in that the NPY Y2 agonist is a peptide containing an amino acid sequence represented by SEQ ID NO: 1.

Further, the NPY Y2 agonist of the present invention is characterized in that the NPY Y2 agonist is a peptide consisting of an amino acid sequence represented by SEQ ID NO: 1.

Further, the NPY Y2 agonist of the present invention is characterized in that the NPY Y2 agonist is a peptide having substitution, deletion, addition or insertion of one or more amino acids in an amino acid sequence represented by SEQ ID NO: 1.

Further, the NPY Y2 agonist of the present invention is characterized in that the disease is diarrhea or irritable bowel syndrome.

Further, the therapeutic agent for a disease accompanied by diarrhea of the present invention is characterized by comprising the above-mentioned NPY Y2 agonist.

Further, the method for evaluating a compound of the present invention is a method for evaluating a compound to be used for the treatment of a disease accompanied by diarrhea, and is characterized by comprising the steps of: preparing a cell expressing NPY Y2 by introducing an NPY Y2 gene; bringing a test compound into contact with the cell; and detecting a specific binding of the test compound to the NPY Y2.

Further, the method for evaluating a compound of the present invention is a method for evaluating a compound to be used for the treatment of a disease accompanied by diarrhea, and is characterized by comprising the steps of: preparing a cell expressing NPY Y2 by introducing an NPY Y2 gene; bringing a test compound into contact with the cell; measuring the activity of an intracellular signal transducer induced by the contact; and comparing the above-mentioned activity with the activity of the intracellular signal transducer in the absence of contact with the test compound.

Further, the method for evaluating a compound of the present invention is a method for evaluating a compound to be used for the treatment of a disease accompanied by diarrhea, and is characterized by comprising the steps of: preparing a cell expressing NPY Y2 by introducing an NPY Y2 gene; bringing a test compound into contact with the cell; measuring the expression level of the NPY Y2 or an intracellular signal transducer mediated by the NPY Y2; and selecting the test compound which increased or decreased the expression level of the NPY Y2 or the intracellular signal transducer mediated by the NPY Y2 in comparison with that before the contact.

Further, the method for evaluating a compound of the present invention is a method for evaluating a compound to be used for the treatment of a disease accompanied by diarrhea, and is characterized by comprising the steps of: bringing a test compound into contact with NPY Y2; and detecting a change in the activity of NPY Y2 caused by the contact.

Further, the method for evaluating a compound of the present invention is characterized in that the compound is an NPY Y2 agonist.

Further, the NPY Y2 ligand of the present invention is characterized in that it is isolated by the above-mentioned method for evaluating a compound.

According to the present invention, an agent capable of inhibiting diarrhea based on a new mechanism of action can be provided. Further, a method for evaluating a compound having an action of inhibiting diarrhea including screening of such an agent can be provided.

### Brief Description of the Drawings

Fig. 1 is a graph showing changes in digestive capacity caused by administration of NPY or PYY3-36.
Fig. 2 is a graph showing changes over time in fecal weight caused by administration of PYY3-36.
Fig. 3 is a graph showing the total fecal weight in 4 hours after administration of PYY3-36.
Fig. 4 is a graph showing changes in fecal weight when any of PYY3-36 and various agents was administered to animal models of diarrhea.
Fig. 5 is a graph showing the total fecal weight in 4 hours after administration of any of PYY3-36 and various agents to animal models of diarrhea.

### Best Mode for Carrying Out the Invention

Hereinafter, a preferred embodiment of the present invention will be described in detail.

The term "NPY Y2" according to the present invention means a molecule having a seven membrane spanning structure that functions as a receptor for NPY as described above. Further, the species from which the NPY Y2 is derived is not particularly limited, and examples thereof include humans, monkeys, mice, rats, dogs and rabbits. In particular, because a subject to which the NPY Y2 agonist of the present invention is administered, or a subject to be evaluated in the evaluation of a compound is a human, it is preferably human NPY Y2.

Further, in the NPY Y2 gene according to the present invention, a gene having substitution, deletion, addition or insertion of one or more nucleotides is also included as long as it has a physiological function equivalent to that of the NPY Y2 gene and encodes a protein that functions as an NPY receptor. Here, the gene is not particularly limited in terms of its sequence as long as it is a gene encoding such a protein. However, it preferably has a homology of 50% or more, more preferably 70% or more, further more preferably 80% or more, and particularly preferably 90% or more (for example, 91, 92, 93, 94, 95, 96, 97, 98, 99% or more).

Further, in the NPY Y2 gene according to the present invention, a nucleic acid which is hybridized to the NPY Y2 gene under a stringent condition is also included. Here, the phrase "which is hybridized under a stringent condition" means that two nucleic acid fragments are hybridized to each other under the hybridization condition described in Molecular Cloning: A Laboratory Manual 2nd Edition, Cold Spring Harbor (1989) 9.47-9.62 and 11.45-11.61. To be more specific, for example, a condition in which washing is carried out at 50°C with 2.0 x SSC after hybridization is carried out at about 45°C with 6.0 x SSC can be exemplified. In order to select the stringency, the salt concentration in the washing step can be selected from about 2.0 x SSC at 50°C as a low stringency to about 0.2 x SSC at 50°C as a high stringency. Further, the temperature in the washing step can be raised from room temperature of about 22°C as a low stringency condition to about 65°C as a high stringency condition.

Further, the "disease accompanied by diarrhea" as used herein is not particularly limited as long as it is a disease or a symptom accompanied by diarrhea, and it does not matter whether it is an acute disease or a chronic disease. Examples of such a disease or symptom include diarrhea (caused by a parasite, a bacterium, a virus, physical stimulation, dyspepsia, mental disorder, impaired gastric acid secretion, gastrointestinal inflammation or allergy, etc.), irritable bowel syndrome, ulcerative colitis and the like.

### (1) NPY Y2 agonist

The "NPY Y2 agonist" as used herein means a substance causing intracellular signal transduction mediated by NPY Y2 receptor and is also referred to as an NPY Y2 stimulating agent. Further, the NPY Y2 agonist according to the present invention is not particularly limited in terms of its molecular species as long as it is a molecule that exhibits an affinity for NPY Y2 receptor and functions as an agonist. Examples of such a molecule include low molecular weight compounds, proteins, and peptides. The low molecular weight compound is also not particularly limited in terms of its type, and specific examples thereof include natural compounds, organic compounds and inorganic compounds. Further, the peptide is also not particularly limited in terms of its type, and specific examples thereof include NPY analogs such as peptide YY (for example, PYY3-36), NPY13-36, [Leu31,Pro34]NPY, and Ac-NPY(24-36) [Leu28,Leu31].

The above-mentioned PYY3-36 (SEQ ID NO: 1) is a peptide which has a homology of 69% with NPY and in which two amino acids at the N-terminus are deleted in PYY (SEQ ID NO: 2), and is known as a molecule that inhibits food intake (Nature, vol. 418, p. 650, 2002). Both PYY3-36 and PYY have a similar activity to that of NPY and have affinity as an agonist for an NPY receptor.

PYY3-36 has an amino acid sequence represented by SEQ ID NO: 1, however, a peptide which has a function as an NPY Y2 agonist and has substitution, deletion, addition or insertion of one or more amino acids in the amino acid sequence is also included in the NPY Y2 agonist of the present invention. The number of amino acids to be substituted, deleted, added or inserted in such a case is not particularly limited as long as the peptide has a function as an NPY Y2 agonist. However, the number of amino acids is preferably 1 to 10, more preferably 1 to 8, further more preferably 1 to 5, and particularly preferably 1 to 3.

As for the NPY Y2 agonist of the present invention, if it is a compound, it can be synthesized by a synthetic method known to a person skilled in the art according to the type of compound. Further, if the compound is derived from a natural product, it can be purified from the natural product by a given extraction method. Further, if the agonist is a peptide, it can be synthesized by a method known to a person skilled in the art.

In the case where the NPY Y2 agonist of the present invention is used as a medicine for humans or other animals, it is possible to administer any of these substances by formulating it into a preparation by a known pharmaceutical method other than the direct administration of the substance as such to a patient. For example, it can be used orally as a tablet, if necessary coated with a sugar, a capsule, an elixir or a microcapsule, or parenterally in the form of an injection of a sterile solution or a suspension with water or a pharmaceutically acceptable liquid other than water. For example, it is possible to formulate the substance into a preparation by appropriately combining a pharmacologically acceptable carrier or medium, specifically sterile water, physiological saline, a vegetable oil, an emulsifier, a suspending agent, a surfactant, a stabilizer, a flavoring agent, an excipient, a vehicle, a preservative, a binder or the like, and mixing them in a unit dosage form as required by generally admitted pharmaceutical practice.

As an additive which can be mixed in a tablet or a capsule, a binder such as gelatin, cornstarch, tragacanth gum or gum arabic, an excipient such as crystalline cellulose, a swelling agent such as cornstarch, gelatin or alginic acid, a lubricant such as magnesium stearate, a sweetener such as sucrose, lactose or saccharine, or a flavoring agent such as peppermint, acamono oil or cherry is used. In the case where the preparation unit is in the capsule form, a liquid carrier such as fat and oil may further be added to the above-mentioned materials. A sterile composition for injection can be formulated according to usual pharmaceutical practice using a vehicle such as distilled water for injection.

Examples of an aqueous solution for injection include physiological saline and isotonic solutions containing glucose or other adjuvants (for example, D-sorbitol, D-mannose, D-mannitol and sodium chloride), and may be used in combination with an appropriate solubilizing agent such as an alcohol, specifically ethanol, a polyalcohol such as propylene glycol or polyethylene glycol or a nonionic surfactant such as Polysolvate 80 (TM) or HCO-50.

Examples of an oily liquid include sesame oil and soybean oil, and may be used in combination with a solubilizing agent such as benzyl benzoate or benzyl alcohol. In addition, it may be further mixed with a buffer such as a phosphate buffer or a sodium acetate buffer, a soothing agent such as procaine hydrochloride, a stabilizer such as benzyl alcohol or phenol, or an antioxidant. The thus prepared injectable solution is usually filled into an appropriate ampoule.

The administration thereof to a patient can be carried out intranasally, transbronchially, intramuscularly, percutaneously or orally by a method known to a person skilled in the art as well as by way of, for example, intraarterial injection, intravenous injection, subcutaneous injection or the like. The dose varies depending on the body weight or age of a patient, an administration route or the like, however, a person skilled in the art can appropriately select a suitable dose. Further, if the agonist can be one encoded by DNA, it is also possible to carry out gene therapy by inserting the DNA into a vector for gene therapy. The dose and administration route vary depending on the body weight, age or symptom of a patient or the like, however, a person skilled in the art can appropriately select them.

The dose of the NPY Y2 agonist varies depending on the symptom, however, in the case of oral administration, the dose thereof is considered to be about 0.1 to 100 mg per day, preferably about 1.0 to 50 mg per day, more preferably about 1.0 to 20 mg per day for generally an adult (assuming of 60 kg of body weight).

In the case of parenteral administration, a single dose thereof varies depending on the subject to be administered, target organ, symptom, or administration route. However, in the case of, for example, an injection, it is considered to be convenient to administer the substance in an amount of generally about 0.01 to 30 mg per day, preferably about 0.1 to 20 mg per day, more preferably about 0.1 to about 10 mg per day by intravenous injection for generally an adult (assuming of 60 kg of body weight).

### (2) Evaluation of compound

A compound that acts on NPY Y2 can be evaluated using an NPY Y2 gene or protein. Examples of the method of detecting an action against NPY Y2 include a method of detecting a specific binding of a test compound to the receptor, a method of detecting the expression level of a gene which is changed by the contact with a test compound and a method of detecting the activity of intracellular signal transduction mediated by NPY Y2 induced by the contact. Hereinafter, these methods will be described in turn.

First, the method for evaluating a test compound by detecting a specific binding of a test compound to the receptor will be described.

The method for evaluating a compound of the present invention is characterized by comprising the steps of: preparing a cell expressing NPY Y2 by introducing an NPY Y2 gene; bringing a test compound into contact with the cell; and detecting a specific binding of the test compound to the NPY Y2.

Further, the second method for evaluating a compound of the present invention is characterized by comprising the steps of: preparing a cell expressing NPY Y2 by introducing an NPY Y2 gene; bringing a test compound into contact with the cell; measuring the activity of an intracellular signal transducer induced by the contact; and comparing the above-mentioned activity with the activity of the intracellular signal transducer in the absence of contact with the test compound.

The test compound is not particularly limited, and examples thereof include single compounds such as natural compounds, organic compounds, inorganic compounds, proteins and peptides, and compound libraries, expression products of gene libraries, cell extracts, cell culture supernatants, fermentation products of microorganisms, marine organism extracts, plant extracts, prokaryotic cell extracts, eukaryotic single cell extracts, animal cell extracts and the like. The above-mentioned test sample can be used by appropriately labeling if necessary. As the labeling, for example, radiolabeling, fluorescent labeling and the like can be exemplified. Further, in addition to the above test samples, a mixture obtained by mixing plural types of these test samples is also included.

Further, the cell expressing an NPY Y2 gene can be prepared by a method known to a person skilled in the art, and a specific method is not particularly limited, and for example, the following method can be employed. That is, it is prepared by cloning an NPY Y2 gene or a nucleic acid consisting of a part thereof into an expression vector containing a suitable promoter and a transcriptional regulatory element, and introducing the vector with the cloned nucleic acid into a host cell. Here, the vector is not particularly limited as long as it is a vector applicable as an expression vector, and examples thereof include pCMV-Tag, pcDNA3.1, pBlueBacHis2, pCI-neo, pcDNAI, pMClneo, pXT1, pSG5, pEFl/V5-HisB, pCR2.1, pET11, λgt11 and pCR3.1.

Subsequently, the expression vector into which the NPY Y2 gene or the nucleic acid consisting of a part thereof is introduced is transfected into a host cell. Such a host cell is not particularly limited as long as it is a host cell commonly used in the expression of a gene, and may be any of an animal cell, an insect cell, a plant cell, and a microorganism. Specific examples thereof include COS1, COS7, CHO, NIH/3T3, 293, Raji, CV11, C1271, MRC-5, CPAE, HeLa, 293T and Sf9. Further, the method of transfecting the expression vector into the host cell is not particularly limited as long as it is a known method, and specific examples thereof include electroporation, the calcium phosphate method, the DEAE-dextran method, the lipofection method and the gene gun method.

Subsequently, the thus prepared cell expressing NPY Y2 is brought into contact with a test compound. The method of contact is not particularly limited, and for example, if NPY Y2 is in a purified state, the contact can be carried out by adding a test sample to a purified preparation. Further, if NPY Y2 is in a state of expressing in a cell (including on a cell membrane) or in a state of expressing in a cell extract, the contact can be carried out by adding a test sample to a cell culture solution or the cell extract, respectively. In the case where a test sample is a protein, for example, a vector containing a DNA encoding the protein is transfected into a cell expressing NPY Y2. Alternatively, the contact can also be carried out by adding the vector to a cell extract in which NPY Y2 is expressed.

The binding of a receptor expressed on a cell surface to a test compound can be detected by, for example, a label attached to the bound compound (for example, detection of binding amount by radioactivity or fluorescence intensity), and other than this, it can be detected using signal transduction into the cell caused by binding of the test compound to the receptor on the cell surface (for example, G protein activation, change in the concentration of cAMP or Ca²⁺ (FLIPR (fluorometric imaging plate reader) or the like can be employed), phospholipase C activation, pH change, or receptor internalization) as an indicator. Further, the expression level or activity of a molecule (also including NPY Y2) involved in a different signal transduction induced by the above-mentioned signal transduction can also be used as an indicator. Here, in the case where the expression level is used as an indicator, the method of measuring the expression level is not particularly limited, and examples thereof include Northern blotting, Western blotting and a DNA chip. Here, the term "expression level" as used herein refers to the absolute amount or relative amount of a transcription product of a gene encoding a protein in the signal transduction pathway mediated by NPY Y2. In this case, in the gene, both DNA and mRNA are included. Further, in the case where the detection target for expression is a protein, the term "expression level" refers to the absolute amount or relative amount of a translation product of a protein in the signal transduction pathway mediated by NPY Y2. Further, in the case where the activity of a molecule involved in signal transduction is used as an indicator, the method of measuring the activity is not particularly limited, and a suitable method may be selected depending on the type of a molecule to be measured.

On the other hand, an isolated NPY Y2 protein can also be used directly for evaluation of a compound. That is, it is a method comprising bringing a test compound into contact with NPY Y2 and detecting a change in the activity of the NPY Y2 protein caused by the contact.

The method of such contact is not particularly limited, and specific examples thereof include a method in which the contact is achieved by mixing in a solution such as a buffer solution (a phosphate buffer solution or the like), and a method in which an NPY Y2 protein is immobilized on a membrane and the protein is brought into contact with a test compound on the membrane.

Subsequently, a change in the activity of NPY Y2 caused by the contact is detected.

The above-mentioned method of measuring the activity of a protein may be appropriately selected depending on the nature of a protein to be used, and specific examples thereof include a method in which the binding activity of a ligand for NPY Y2 is used as an indicator.

The method in which the binding activity of a ligand is used as an indicator is not particularly limited, and specific examples thereof include a method in which the binding activity is determined by measuring affinity of a test compound for a membrane on which NPY Y2 is immobilized. The test compound used here may be labeled with a radioisotope or the like so as to facilitate the detection. Further, as the method of detecting the binding activity, a method of detecting a compound that binds to NPY Y2 in a manner competitive with a ligand labeled with a radioisotope can be exemplified. In the case where such a method is used, the test compound does not need to be labeled.

As described above, in the case where as a result of detecting a compound by the method for evaluating a compound of the present invention, the binding activity in the presence of a test compound has a lower value than the binding activity in the absence of the test compound (control), the test compound is judged to have an activity of inhibiting the binding between the NPY Y2 according to the present invention and the ligand. Such compounds include compounds which have an activity of inducing signal transduction into a cell through binding to the receptor (agonists), compounds which do not have such an activity (antagonists) and the like. An agonist has the same physiological activity as the ligand for the receptor and its analogs, while an antagonist inhibits the physiological activity of the ligand for the receptor and its analogs. Thus, such agonists and antagonists are useful as a pharmaceutical composition for the treatment and the like of a disease caused by abnormalities in signal transduction systems mediated by NPY Y2 according to the present invention.

Further, by the method for evaluating a compound of the present invention, screening of a substance that promotes or inhibits intracellular signal transduction after binding of a test compound to NPY Y2. That is, by evaluating multiple test compounds by the above-mentioned method, a compound that functions as an agonist or an antagonist can be selected. If, as a result of such selection, in comparison with the change in intracellular signal transduction in the case where the ligand or its analog is allowed to act in the absence of the test compound, the change is suppressed, the test compound is judged to be a compound that inhibits the intracellular signal transduction after binding of the test compound to NPY Y2. On the contrary, if the test compound enhances intracellular signal transduction, the compound is judged to be a compound that promotes the intracellular signal transduction after binding of the test compound to NPY Y2. A compound selected by such a screening method is effective in the treatment of a disease accompanied by diarrhea or the alleviation of the symptom, and above all, a compound that functions as an NPY Y2 agonist is particularly effective in the treatment of a disease accompanied by diarrhea or the alleviation of the symptom.

### (Examples)

Hereinafter, the present invention will be described further specifically with reference to Examples, however, the present invention is not limited to the following Examples.

### (Example 1)

### (Study of motility of stomach and small intestine by administration of NPY Y2 agonist)

In order to study a change in the motility of stomach and small intestine by administration of an NPY Y2 agonist, the following test was carried out.

First, mice (C57BL/6) to be used in the test were prepared. Mice were individually housed in cages with a floor mat in an animal room with a controlled environment (temperature: 23°C, relative humidity: 55%, lighting hours: from 7:00 till 19:00) with ad libitum access to rodent chow (CE-2, Nippon CLEA) and drinking water.

Subsequently, NPY (0.3 mg/kg or 3 mg/kg) or PYY3-36 (0.3 mg/kg or 3 mg/kg) was intraperitoneally administered to the mice, and the motility of stomach and small intestine was measured. At the same time, control mice (vehicle) to which only a vehicle was administered were prepared, and the same test was carried out using these mice. The motility of stomach and small intestine was measured as follows. That is, charcoal powder was suspended in a 0.5% methyl cellulose solution and the resulting suspension was orally administered to the mice. After 30 minutes, the distance that the charcoal entering into the stomach traveled in the intestinal tract was measured by dissection. At the same time, the total length of the small intestine from the pyloric portion of stomach to the beginning of the cecum was also measured, and the ratio (%) of the distance that the charcoal traveled to the total length of the small intestine was plotted in a graph as a GI (gastrointestinal) motility.

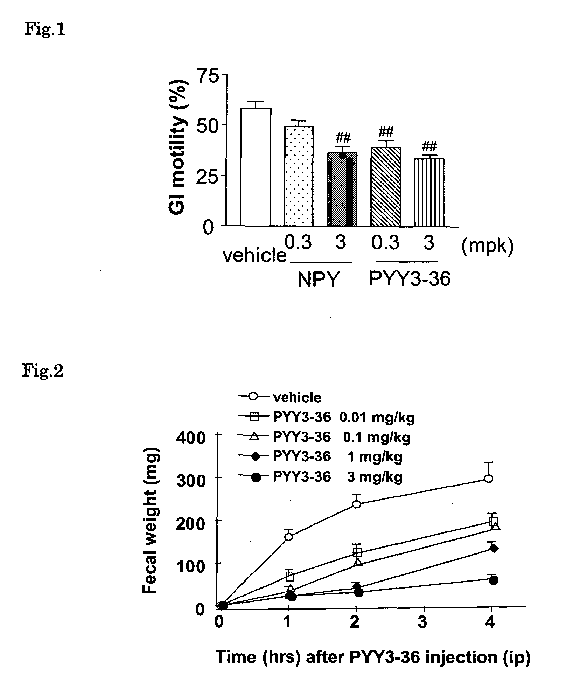

As shown in Fig. 1, it was found that the GI motility of the mice to which NPY or PYY3-36 was administered is lower than that of the control mice. That is, it could be confirmed that the motility of stomach and small intestine was inhibited by the administration of an NPY Y2 agonist.

### (Example 2)

### (Study of change in fecal weight as indicator for colonic motility by administration of NPY Y2 agonist)

In order to study a change in the fecal weight as an indicator for colonic motility by administration of an NPY Y2 agonist, the following test was carried out.

In the test, mice (C57BL/6) were used, and they were housed and fed in the same manner as in Example 1.

Subsequently, PYY3-36 (3 mg/kg) was intraperitoneally administered to the mice, and the fecal weight was measured in a time-dependent manner (1, 2, 3, and 4 hours after the administration). At the same time, the fecal weight was also measured for control mice to which only a vehicle was administered.

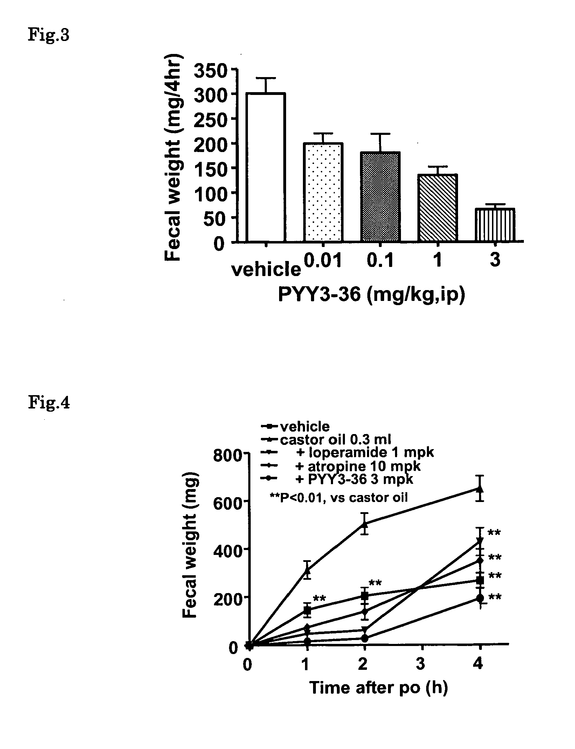

As shown in Fig. 2, a difference in the fecal weight already began to be observed at 1 hour after the administration, and also the fecal weight thereafter of the mice to which PYY3-36 was administered was significantly lower than that of the control mice. Further, as shown in Fig. 3, as also for the total fecal weight in 4 hours after the administration of PYY3-36, a significant difference was observed compared with that of the control mice. That is, it could be confirmed that the colonic motility was inhibited by the administration of PYY3-36, whereby defecation was inhibited.

### (Examples 3 and Comparative examples 1 and 2)

### (Study of effect of administration of NPY Y2 agonist on castor oil-induced defecation and diarrheal symptom)

In order to study an inhibitory effect of administration of an NPY Y2 agonist on castor oil-induced defecation and diarrheal symptom, the following test was carried out.

In the test, mice (C57BL/6) were used, and they were housed and fed in the same manner as in Example 1.

Subsequently, each of PYY3-36 (3 mg/kg) and other test agents (loperamide (1 mg/kg) (Comparative example 1) and atropine (10 mg/kg) (Comparative example 2)) was intraperitoneally administered to the mice, and at 15 minutes after the administration, castor oil (0.3 ml/animal) was orally administered to the mice. The fecal weight and diarrhea score (normal: 0, soft feces: 1, watery feces: 2) were measured in a time-dependent manner (1, 2, and 4 hours after the administration). At the same time, the same measurement was carried out for mice to which only a vehicle was administered and castor oil was not administered.

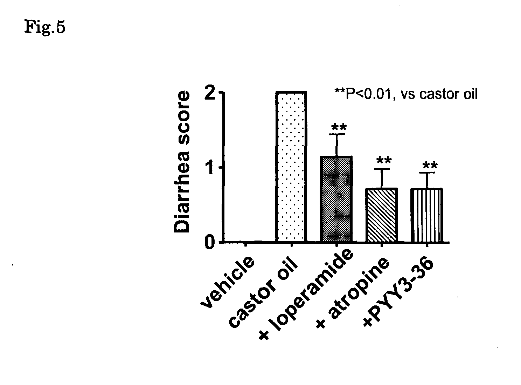

As shown in Fig. 4, in the mice pre-administered with PYY3-36, the inhibition of fecal weight already occurred at 1 hour after the treatment with castor oil, and also the fecal weight thereafter was significantly lower than that of the control mice treated with the vehicle and castor oil. Further, as shown in Fig. 5, the maximum diarrhea score observed until 4 hours after the treatment with castor oil was significantly inhibited in the mice pre-administered with PYY3-36 compared with the control mice treated with the vehicle and castor oil. That is, it could be confirmed that the colonic motility was inhibited by the administration of PYY3-36, whereby the diarrheal symptom was inhibited. Further, even in the comparison with conventional antidiarrheal agents, loperamide and atropine, a high inhibitory effect of PYY3-36 on diarrheal symptom was observed.

### Industrial Applicability

As described above, with the use of the NPY Y2 agonist and the method for evaluating a compound of the present invention, an agent capable of inhibiting diarrhea based on a new mechanism of action can be provided. Further, a method for evaluating a compound having an action of inhibiting diarrhea including screening of such an agent can be provided.

## Claims

1. An NPY Y2 agonist, **characterized in that** it is a therapeutic agent for a disease accompanied by diarrhea.

2. The NPY Y2 agonist according to claim 1, **characterized in that** the NPY Y2 agonist is an NPY analog.

3. The NPY Y2 agonist according to claim 1 or 2, **characterized in that** the NPY Y2 agonist is a peptide containing an amino acid sequence represented by SEQ ID NO: 1.

4. The NPY Y2 agonist according to claim 1 or 2, **characterized in that** the NPY Y2 agonist is a peptide consisting of an amino acid sequence represented by SEQ ID NO: 1.

5. The NPY Y2 agonist according to claim 1 or 2, **characterized in that** the NPY Y2 agonist is a peptide having substitution, deletion, addition or insertion of one or more amino acids in an amino acid sequence represented by SEQ ID NO: 1.

6. The NPY Y2 agonist according to any one of claims 1 to 5, **characterized in that** the disease is diarrhea or irritable bowel syndrome.

7. A therapeutic agent for a disease accompanied by diarrhea, **characterized by** comprising the NPY Y2 agonist according to any one of claims 1 to 6.

8. A method for evaluating a compound to be used for the treatment of a disease accompanied by diarrhea, **characterized by** comprising the steps of:
preparing a cell expressing NPY Y2 by introducing an NPY Y2 gene;
bringing a test compound into contact with the cell; and
detecting a specific binding of the test compound to the NPY Y2.

9. A method for evaluating a compound to be used for the treatment of a disease accompanied by diarrhea, **characterized by** comprising the steps of:
preparing a cell expressing NPY Y2 by introducing an NPY Y2 gene;
bringing a test compound into contact with the cell;
measuring the activity of an intracellular signal transducer induced by the contact; and
comparing said activity with the activity of the intracellular signal transducer in the absence of contact with the test compound.

10. A method for evaluating a compound to be used for the treatment of a disease accompanied by diarrhea, **characterized by** comprising the steps of:
preparing a cell expressing NPY Y2 by introducing an NPY Y2 gene;
bringing a test compound into contact with the cell;
measuring the expression level of the NPY Y2 or an intracellular signal transducer mediated by the NPY Y2; and
selecting the test compound which increased or decreased the expression level of the NPY Y2 or the intracellular signal transducer mediated by the NPY Y2 in comparison with that before the contact.

11. A method for evaluating a compound to be used for the treatment of a disease accompanied by diarrhea, **characterized by** comprising the steps of:
bringing a test compound into contact with NPY Y2; and
detecting a change in the activity of NPY Y2 caused by the contact.

12. The method for evaluating a compound according to any one of claims 8 to 11, **characterized in that** the compound is an NPY Y2 agonist.

13. An NPY Y2 ligand, **characterized in that** it is isolated by the method for evaluating a compound according to any one of claims 8 to 12.


SEQUENCE LISTING

<110>  BANYU Pharmaceutical co., ltd.

<120>  NPY Y2 Agonist as medicine of disorder with diarrhea

<130>  BY0075

<150>  JP2005-169851
<151>  2005-06-09

<160>  1

<170>  PatentIn version 3.1

<210>  1
<211>  34
<212>  PRT
<213>  Homo sapiens

<400>  1

Ile Lys Pro Glu Ala Pro Gly Glu Asp Ala Ser Pro Glu Glu Leu Asn
1               5                   10                  15


Arg Tyr Tyr Ala Ser Leu Arg His Tyr Leu Asn Leu Val Thr Arg Gln
            20                  25                  30


Arg Tyr